(19)

Europäisches Patentamt

European Patent Office

Office européen des brevets

(11) **EP 4 451 182 A2**

(12) **EUROPEAN PATENT APPLICATION**

(43) Date of publication:
**23.10.2024 Bulletin 2024/43**

(21) Application number: **24176918.1**

(22) Date of filing: **17.07.2017**

(51) International Patent Classification (IPC):
*G06N 20/20* (2019.01)

(52) Cooperative Patent Classification (CPC):
**G06N 20/20; A61B 5/0205; A61B 5/7267;
G06N 20/00; G16H 50/20; G16H 50/70;** G06N 3/08;
G06N 5/01; G06N 20/10

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.07.2016  US 201662365880 P
13.07.2017  US 201715649489**

(62) Document number(s) of the earlier application(s) in
accordance with Art. 76 EPC:
**17831627.9 / 3 488 370**

(71) Applicant: **Edwards Lifesciences Corporation
Irvine, CA 92614 (US)**

(72) Inventors:
• **Al Hatib, Feras
Irvine, CA 92614 (US)**

• **Jian, Zhongping
Irvine, CA 92614 (US)**

(74) Representative: **Eisenführ Speiser
Patentanwälte Rechtsanwälte PartGmbB
Gollierstraße 4
80339 München (DE)**

Remarks:
•This application was filed on 21-05-2024 as a
divisional application to the application mentioned
under INID code 62.
•claims filed after the date of filing of the application
/ after the date of receipt of the divisional application
(Rule 68(4) EPC)

(54) **PREDICTIVE RISK MODEL OPTIMIZATION**

(57)     A system disclosed herein includes a hardware processor and a predictive risk model training software code stored in a system memory. The hardware processor executes the software code to receive vital sign data of a population of subjects including positive and negative subjects with respect to a health state, to define data sets for use in training a predictive risk model, to transform the vital sign data to parameters characterizing the vital sign data, and to obtain differential parameters based on those parameters. The hardware processor executes the software code to further generate combinatorial parameters using the parameters and the differential parameters, to analyze the parameters, the differential parameters, and the combinatorial parameters to identify a reduced set of parameters correlated with the health state, to identify a predictive set of parameters enabling prediction of the health state for a living subject, and to compute predictive risk model coefficients.

Fig. 1

EP 4 451 182 A2

**Description**

BACKGROUND

Critically ill patients and patients undergoing surgery are at risk of entering a number of serious physiological states that, if not promptly detected and effectively treated, can lead to irreversible organ damage, and even death. Examples of such physiological states include hypotension, hypovolemia, acute blood loss, septic shock, and cardiovascular collapse or "crash," to name a few. For each of these physiological states, the earliest possible detection is crucial in order to prevent permanent injury to the affected patient. Even more advantageous would be the ability to predict onset of some or all of these physiological states in order to prepare an appropriate medical intervention in advance.

As a specific example, hypotension, or low blood pressure, can be a harbinger of grave medical complications for patients undergoing surgery and for critically ill patients receiving treatment in an intensive care unit (ICU). In the operating room (OR) setting, hypotension during surgery is associated with increased mortality and organ injury. Moreover, hypotension is relatively common, and is often seen as one of the first signs of patient deterioration in the OR and ICU. For instance, hypotension is seen in up to approximately thirty-three percent of surgeries overall, and up to eighty-five percent in high risk surgeries. Among ICU patients, hypotension occurs in from approximately twenty-four percent to approximately eighty-five percent of all patients, with the eighty-five percent occurrence being seen among critically ill patients.

Conventional patient monitoring for hypotension and the other serious physiological states described above can include continuous or periodic measurement of vital signs, such as blood pressure, pulse rate, respiration, and the like. However, such monitoring, whether performed continuously or periodically, typically provides no more than a real-time assessment of the patient's condition. As a result, hypotension and the other serious physiological states described above are usually detected only after their onset, so that remedial measures and interventions cannot be initiated until the patient has begun to deteriorate. However, these physiological states can have potentially devastating medical consequences quite quickly. For example, even relatively mild levels of hypotension can herald or precipitate cardiac arrest in patients with limited cardiac reserve.

In view of the susceptibility of OR and ICU patients to hypotension and other potentially dangerous physiological states, and due to the serious and sometimes immediate medical consequences that can result when a patient enters those states, a solution enabling prediction of future patient deterioration due to hypotension, hypovolemia, acute blood loss, or crash, for example, is highly desirable.

SUMMARY

There are provided systems and methods for predictive risk model optimization, substantially as shown in and/or described in connection with at least one of the figures, and as set forth more completely in the claims.

BRIEF DESCRIPTION OF THE DRAWINGS

Figure 1 shows a diagram of an exemplary system for training a predictive risk model, according to one implementation of the present disclosure;
Figure 2 shows another exemplary implementation of a system for training a predictive risk model;
Figure 3 shows an exemplary system and a computer-readable non-transitory medium including instructions for performing predictive risk model training, according to one implementation; and
Figure 4 is a flowchart presenting an exemplary method for use by a system for training a predictive risk model, according to one implementation.

DETAILED DESCRIPTION

The following description contains specific information pertaining to implementations in the present disclosure. One skilled in the art will recognize that the present disclosure may be implemented in a manner different from that specifically discussed herein. The drawings in the present application and their accompanying detailed description are directed to merely exemplary implementations. Unless noted otherwise, like or corresponding elements among the figures may be indicated by like or corresponding reference numerals. Moreover, the drawings and illustrations in the present application are generally not to scale, and are not intended to correspond to actual relative dimensions.

The present application is directed to systems and methods for providing improved patient care through predictive risk modeling for a variety of potentially dangerous physiological states to which a critically ill or surgical patient may be susceptible. To that end, the present application discloses systems and methods for training a predictive risk model so as to substantially optimize the reliability with which the model can predict the advent of such a dangerous physiological state in a patient. Examples of those dangerous physiological states include hypotension, hypovolemia, acute blood loss, septic shock, and cardiovascular collapse or "crash," to name a few. According to various implementations, the systems and methods disclosed in the present application may be utilized by health care workers to anticipate a dangerous physiological state prior to its onset. As a result of such forewarning, the systems and methods disclosed in the present application enable preparation of effective medical interventions for administering early treatment of the anticipated condition, or for preventing it entirely.

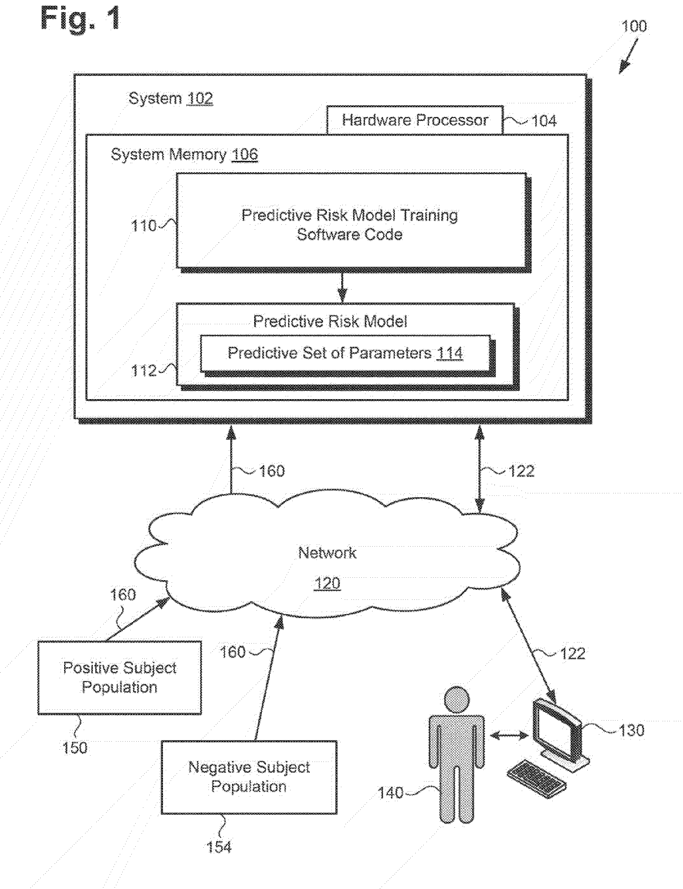

Figure 1 shows a diagram of an exemplary system for training a predictive risk model, according to one implementation. As shown in Figure 1, system 102 is situated within communication environment 100 including communication network 120, client system 130, system user 140, positive subject population 150, and negative subject population 154.

System 102 includes hardware processor 104, and system memory 106 storing predictive risk model training software code 110. In addition, system memory 106 is shown to include predictive risk model 112 including predictive set of parameters 114. Also shown in Figure 1 are network communication links 122 interactively connecting client system 130 and system 102 via communication network 120, as well as vital sign data 160 received by system 102 from positive subject population 150 and negative subject population 154 via communication network 120.

According to the implementation shown in Figure 1, system user 140, who may be a health care worker or medical researcher, for example, may utilize client system 130 to interact with system 102 over communication network 120. For instance, system user 140 may receive predictive risk model 112 including predictive set of parameters 114 over communication network 120, and/or may download predictive risk model training software code 110 to client system 130, via communication network 120. In one implementation, system 102 may correspond to one or more web servers, accessible over a packet network such as the Internet, for example. Alternatively, system 102 may correspond to one or more servers supporting a local area network (LAN), or included in another type of limited distribution network.

Hardware processor 104 is configured to execute predictive risk model training software code 110 to receive vital sign data 160 of each subject of a population of subjects including positive subject population 150 and negative subject population 154 with respect to a health state. For example, in one exemplary implementation, the health state may be hypotension, with positive subject population 150 including only subjects having experienced hypotension, and negative subject population 150 including only subjects who have not. In that implementation, for example, vital sign data 160 may include arterial pressure data for each subject.

Hardware processor 104 is further configured to execute predictive risk model training software code 110 to transform vital sign data 160 to multiple parameters characterizing vital sign data 160. In addition, hardware processor 104 is configured to execute predictive risk model training software code 110 to obtain differential parameters based on the multiple parameters characterizing vital sign data 160, and to generate combinatorial parameters using the multiple parameters characterizing vital sign data 160 and the differential parameters. Hardware processor 104 is also configured to execute predictive risk model training software code 110 to analyze the multiple parameters characterizing vital sign data 160, the differential parameters, and the combinatorial parameters to identify a reduced set of parameters correlated with the health state. Hardware processor 104 is further configured to execute predictive risk model training software code 110 to identify, from among the reduced set of parameters, predictive set of parameters 114 enabling prediction of the health state for a living subject, thereby training predictive risk model 112 so as to substantially optimize its predictive reliability.

In some implementations, hardware processor 104 is configured to execute predictive risk model training software code 110 to display predictive risk model 112, and/or parameters characterizing vital sign data 160, and/or predictive set of parameters 114, to system user 140, through display features available on client system 130, for example. In some implementations, hardware processor 104 is configured to execute predictive risk model training software code 110 to update or otherwise modify predictive set of parameters 114 based on additional vital sign data 160 received from one or more of positive subject population database 150 and negative subject population database 154.

It is noted that although Figure 1 depicts predictive risk model 112 as residing in system memory 106, in some implementations, predictive risk model 112 may be copied to non-volatile storage (not shown in Figure 1), or may be transmitted to client system 130 via communication network 120 as mentioned above. It is further noted that although client system 130 is shown as a personal computer (PC) in Figure 1, that representation is provided merely as an example. In other implementations, client system 130 may be a mobile communication device, such as a smartphone or tablet computer, for example.

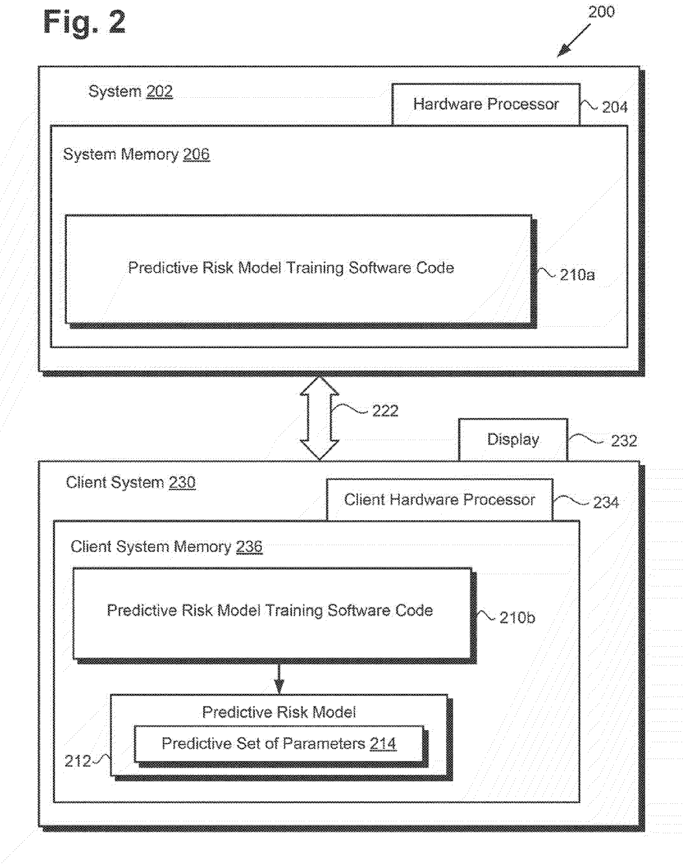

Referring to Figure 2, Figure 2 shows a more detailed exemplary implementation of client system 230, which may itself be configured to train a predictive risk model. Communication environment 200 in Figure 2 includes client system 230 interactively connected to system 202 over network communication link 222. As shown in Figure 2, system 202 includes hardware processor 204, and system memory 206 storing predictive risk model training software code 210a. As further shown in Figure 2, client system 230 includes display 232, client hardware processor 234, and client system memory 236 storing predictive risk model training software code 210b. Also shown in Figure 2 is predictive risk model 212 including predictive set of parameters 214.

Network communication link 222, and system 202 including hardware processor 204 and system memory 206 correspond in general to network communication link 122, and system 102 including hardware processor 104 and system memory 106, in Figure 1. In addition, predictive risk model training software code 210a, in Figure 2, corresponds to predictive risk model training software code 110, in Figure 1. In other words, predictive risk model training software code 210a may share any of the characteristics attributed to corresponding predictive risk model training software code 110, in Figure 1, as described in the present application.

Client system 230 corresponds in general to client system 130, in Figure 1. Moreover, predictive risk model training software code 210b corresponds to predictive risk model training software code 110/210a. As a result, predictive risk model training software code 210b and predictive risk model 212 including predictive set of parameters 214 may share any of the characteristics attributed to corresponding predictive risk model training software code 110 and predictive risk model 112 including predictive set of parameters 114 shown in Figure 1, as described in the present application.

According to the exemplary implementation shown in Figure 2, predictive risk model training software code 210b is located in client system memory 236, having been received from system 202 via network communication link 222. In one implementation, network communication link 222 corresponds to transfer of predictive risk model training software code 210b over a packet network, for example. Once transferred, for instance by being downloaded over network communication link 222, predictive risk model training software code 210b may be persistently stored in client system memory 236 and may be executed locally on client system 230 by client hardware processor 234.

Client hardware processor 234 may be the central processing unit (CPU) for client system 230, for example, in which role client hardware processor 234 runs the operating system for client system 230 and executes predictive risk model training software code 210b. In the exemplary implementation of Figure 2, a user of client system 230, such as system user 140, in Figure 1, can utilize predictive risk model training software code 210b on client system 230 to identify predictive set of parameters 214, thereby training predictive risk model 212.

In addition, system user 140 can utilize predictive risk model training software code 210b on client system 230 to display predictive risk model 212, and/or parameters characterizing vital sign data 160, and/or predictive set of parameters 214, on display 232. Display 232 may take the form of a liquid crystal display (LCD), a light-emitting diode (LED) display, an organic light-emitting diode (OLED) display, or another suitable display screen that performs a physical transformation of signals to light so as to display predictive risk model 212, and/or parameters characterizing vital sign data 160, and/or predictive set of parameters 214, to system user 140.

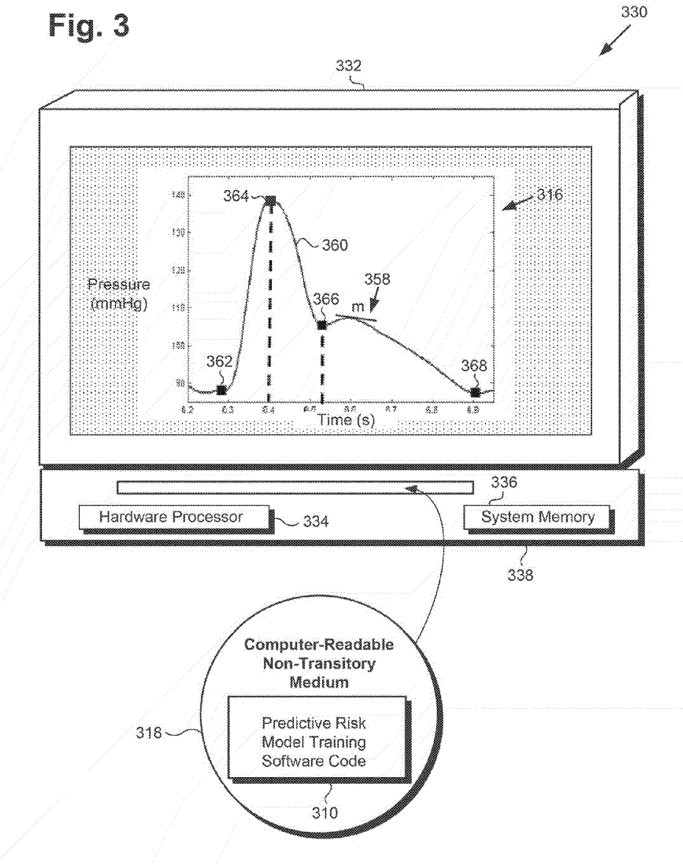

Moving now to Figure 3, Figure 3 shows an exemplary system and a computer-readable non-transitory medium including instructions enabling predictive risk model training and substantial optimization, according to one implementation. System 330 includes computer 338 having hardware processor 334 and system memory 336, interactively linked to display 332. Like display 232, in Figure 2, display 332 may take the form of an LCD, LED, or OLED display, for example, configured to perform a physical transformation of signals to light so as to display, for example, parameters 316 characterizing vital sign data 160. System 330 including hardware processor 334 and system memory 336 corresponds in general to any or all of system 102 and client system 130, in Figure 1, and system 202 and client system 230, in Figure 2.

It is noted that parameters 316 characterizing vital sign data 160 are shown on display 332 and include features 362, 364, 366, 368, and 358 of arterial pressure waveform 360. Features 362, 364, 366, 368, and 358 included among parameters 316 correspond respectively to the start of the heartbeat producing arterial pressure waveform 360, the maximum systolic pressure marking the end of systolic rise, the presence of the dicrotic notch marking the end of systolic decay, the diastole of the heartbeat, and an exemplary slope of arterial pressure waveform 360. It is further noted that parameters 316 including arterial pressure waveform 360 and features 362, 364, 366, 368, and 358 can correspond to a specific case in which the health state for which predictive risk model generation is being performed is hypotension.

In addition to the features 362, 364, 366, and 368 of arterial pressure waveform 360 per se, the behavior of arterial pressure waveform 360 during the intervals between those features may also be used as parameters characterizing vital sign data 160. For example, the interval between the start of the heartbeat at feature 362 and the maximum systolic pressure at feature 364 marks the duration of the systolic rise (hereinafter "systolic rise 362-364"). The systolic decay of arterial pressure waveform 360 is marked by the interval between the maximum systolic pressure at feature 364 and the dicrotic notch at feature 366 (hereinafter "systolic decay 364-366"). Together, systolic rise 362-364 and systolic decay 364-366 mark the entire systolic phase (hereinafter "systolic phase 362-366"), while the interval between the dicrotic notch at feature 366 and the diastole at feature 368 mark the diastolic phase of arterial pressure waveform 360 (hereinafter "diastolic phase 366-368").

Also of potential diagnostic interest is the behavior of arterial pressure waveform 360 in the interval from the maximum systolic pressure at feature 364 to the diastole at feature 368 (hereinafter "interval 364-368"), as well as the behavior of arterial pressure waveform 360 from the start of the heartbeat at feature 362 to the diastole at feature 368 (hereinafter "heartbeat interval 362-368"). The behavior of arterial pressure waveform 360 during intervals: 1) systolic rise 362-364, 2) systolic decay 364-366, 3) systolic phase 362-366, 4) diastolic phase 366-368, 5) interval 364-368, and 6) heartbeat interval 362-368 may be determined by measuring the area under the curve of arterial pressure waveform 360 and the standard deviation of arterial pressure waveform 360 in each of those intervals, for example. The respective areas and standard deviations measured for intervals 1, 2, 3, 4, 5, and 6 above (hereinafter "intervals 1-6") may serve as additional parameters characterizing vital sign data 160.

Also shown in Figure 3 is computer-readable non-transitory medium 318 having predictive risk model training software code 310 stored thereon. The expression "computer-readable non-transitory medium," as used in the present application, refers to any medium, excluding a carrier wave or other transitory signal, that provides instructions to hardware processor 334 of computer 338. Thus, a computer-readable non-transitory medium may correspond to various types of media, such as volatile media and non-volatile media, for example. Volatile media may include dynamic memory, such as dynamic random access memory (dynamic RAM), while non-volatile memory may include optical, magnetic, or electrostatic storage devices. Common forms of computer-readable non-transitory media include, for example, optical discs, RAM, programmable read-only memory (PROM), erasable PROM (EPROM), and FLASH memory.

According to the implementation shown in Figure 3, computer-readable non-transitory medium 318 provides predictive risk model training software code 310 for execution by hardware processor 334 of computer 338. Predictive risk model training software code 310, when executed by hardware processor 334, instantiates a predictive risk model training software code corresponding to predictive risk model training software code 110/210a/210b, in Figure 1/2, and capable of performing all of the operations attributed to that corresponding feature by the present application. For example, predictive risk model training software code 310, when executed by hardware processor 334, is configured to identify predictive set of parameters 114/214, thereby training predictive risk model 112/212.

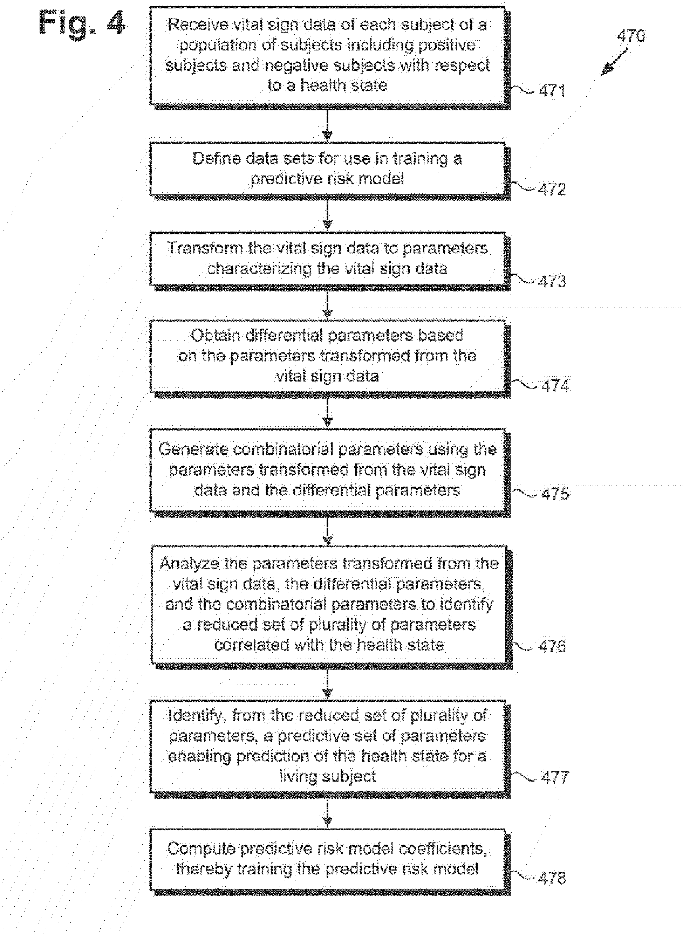

The systems for training predictive risk models discussed above by reference to Figures 1, 2, and 3, will be further described below with reference to Figure 4. Figure 4 presents flowchart 470 outlining an exemplary method for use by a system for training a predictive risk model, according to one implementation. The method outlined in flowchart 470 can be performed using predictive risk model training software code 110/210a/210b/310, executed by hardware processor 104/204/234/334.

Flowchart 470 begins with receiving vital sign data 160 of each subject of a population of subjects including positive subject population 150 and negative subject population 154 with respect to a health state (action 471). Vital sign data 160 may be received by predictive risk model training software code 110/210a/210b/310 of system 102/202/230/330, executed by hardware processor 104/204/234/334. As shown in Figure 1, vital sign data 160 may be received by predictive risk model training software code 110/210a/210b/310 from positive subject population 150 and negative subject population 154 via communication network 120.

It is noted that in the interests of conceptual clarity, the method outlined by flowchart 470 will be described with reference to a specific implementation in which the health state for which a predictive risk model is being trained so as to be substantially optimized is hypotension. However, it is emphasized that the systems and methods disclosed by the present application can be adapted to perform predictive risk model training and substantial optimization for other health states of interest, such as hypovolemia, acute blood loss, sepsis or septic shock, extubation failure, post-surgical complications, and cardiovascular collapse or crash, for example. With respect to the specific and exemplary case in which the health state of interest is hypotension, vital sign data 160 may take the form of hemodynamic data corresponding to the arterial pressure of each subject of positive subject population 150 and negative subject population 154.

Flowchart 470 continues with defining data subsets for use in the training of predictive risk model 112/212 (hereinafter "training subsets") (action 472). The training subsets may be obtained from vital sign data 160 of positive subject population 150 and negative subject population 154 with respect to a health state, e.g., in the present example, hypotension. The positive training subset may be defined as all the periods of time when the health state occurred in positive subject population 150. The positive training subset may also be defined as all the periods of time when the health state occurred, as well as periods of times prior to the occurrence of the health state (example 5, 10 or 15 minutes prior to the occurrence of the health state) in positive subject population 150. An example of a positive training subset is all the data points when a hypotensive event occurred, as well as all data points 5, 10, or 15 minutes prior to the hypotensive event.

The negative training subset may be defined as all the periods of time when the health state did not occur in negative subject population 154. The negative training subset may also be defined in positive subject population 150 as all the periods of time when the health state did not occur, where periods of time must be at some time distance removed from the period of time when the health state occurred. An example of a negative training subset is all the data points when a hypotensive event did not occur, and the periods of time must be at least 20 minutes away (before and after) from the closest hypotensive event. The negative training subset may also be defined as all data points for negative subject population 154.

Flowchart 470 continues with transforming vital sign data 160 to parameters 316 characterizing vital sign data 160 (action 473). Transformation of vital sign data 160 to parameters 316 may be performed by predictive risk model training software code 110/210a/210b/310, executed by hardware processor 104/204/234/334. As discussed above with reference to Figure 3, parameters 316 can include features of arterial pressure waveform 360 including the start 362 of the heartbeat producing arterial pressure waveform 360, the maximum systolic pressure 364 marking the end of systolic rise, the presence of the dicrotic notch 364 marking the end of systolic decay, the diastole 368 of the heartbeat, and exemplary slope 358 of arterial pressure waveform 360.

In addition, parameters 316 may further include the respective areas and standard deviations measured for intervals 1-6 of arterial pressure waveform 360, as discussed above by reference to Figure 3. Arterial pressure waveform 360 may be a central arterial pressure waveform of any subject from positive subject population 150 or negative subject population 154, for example.

It is noted that slope 358 is merely representative of multiple slopes that may be measured at multiple locations along arterial pressure waveform 360. It is further noted that parameters 316 provide a mere sampling of the parameters which may be transformed from vital sign data 160. In practice, parameters 316 may include hundreds of parameters. Examples of additional parameters that might typically be included among parameters 316 are cardiac output, cardiac index, stroke volume, stroke volume index, pulse rate, systemic vascular resistance, systemic vascular resistance index, and mean arterial pressure (MAP). In addition, parameters 316 may include a variety of different types of parameters found to be predictive of future hypotension. For instance, parameters 316 may include any or all of baroreflex sensitivity measures, hemodynamic complexity measures, and frequency domain hemodynamic features.

Baroreflex sensitivity measures quantify the relationship between complementary physiological processes. For example, a decrease in blood pressure in a healthy living subject is typically compensated by an increase in heart rate and/or an increase in peripheral resistance. The baroreflex sensitivity measures that may be derived from arterial pressure waveform 360 correspond to the degree to which the subject producing arterial pressure waveform 360 responded appropriately to normal physiological variations. Hemodynamic complexity measures quantify the amount of regularity in cardiac measurements over time, as well as the entropy, i.e., the unpredictability of fluctuations in cardiac measurements. Frequency domain hemodynamic features quantify measures of cardiac performance as a function of frequency rather than time.

Flowchart 470 continues with obtaining differential parameters based on parameters 316 (action 474). Obtaining differential parameters based on parameters 316 (hereinafter "the differential parameters") may be performed by predictive risk model training software code 110/210a/210b/310, executed by hardware processor 104/204/234/334. The differential parameters may be obtained by determining the variations of parameters 316 with respect to time, with respect to frequency, or with respect to other parameters from among parameters 316, for example. As a result, each of parameters 316 may give rise to one, two, or several differential parameters.

For example, the differential parameter stroke volume variation (SVV) may be obtained based on changes in the parameter stroke volume (SV) as a function of time and/or as a function of sampling frequency. Analogously, changes in mean arterial pressure ($\Delta$MAP) can be obtained as a differential parameter with respect to time and/or sampling frequency, and so forth. As a further example, changes in mean arterial pressure with respect to time can be obtained by subtracting the average of the mean arterial pressure over the past 5 minutes, over the past 10 minutes, and so on from the current value of the mean arterial pressure. As noted above, parameters 316 may number in the hundreds, while one or more differential parameters may be obtained from each of parameters 316. As a result, parameters 316 and the differential parameters, together, can number in the thousands.

Flowchart 470 continues with generating combinatorial parameters using parameters 316 and the differential parameters (action 475). Generation of such combinatorial parameters may be performed by predictive risk model training software code 110/210a/210b/310, executed by hardware processor 104/204/234/334. For example, a combinatorial parameter may be generated using parameters 316 and the differential parameters by generating a power combination of a subset of parameters 316 and the differential parameters. It is noted that, as used in the present application, the characterization "a subset of parameters 316 and the differential parameters" refers to a subset of parameters fewer in number than parameters 316 and fewer in number than the differential parameters, and which includes some of parameters 316 and/or some of the differential parameters.

As a specific example, each of the combinatorial parameters may be generated as a power combination of three parameters, which may be randomly or purposefully selected, from among parameters 316 and/or the differential parameters. Each of those three parameters selected from among parameters 316 and/or the differential parameters can be raised to an exponential power and can be multiplied with, or added to, the other two parameters analogously raised to an exponential power. The exponential power to which each of the three parameters selected from parameters 316 and/or the differential parameters is raised may be, but need not be, the same.

In some implementations, for example, generation of the combinatorial parameters may be performed using a predetermined and limited integer range of exponential powers. For instance, in one such implementation, the exponential powers used to generate the combinatorial parameters may be integer powers selected from among negative two, negative one, zero, one, and two (-2, -1, 0, 1, 2). Thus, each combinatorial parameter may take the form:

$$X = Y_1{}^a * Y_2{}^b * \ldots Y_n{}^c \qquad \text{(Equation 1)}$$

where each Y is one of parameters 316 or one of the differential parameters, n is any integer greater than two, and each of a, b, and c may be any one of -2, -1, 0, 1, and 2, for example. In one implementation, Equation 1 may be applied to substantially all possible power combinations of parameters 316, the differential parameters, and parameters 316 with the differential parameters, subject to the predetermined constraints discussed above, such as the value of n and the numerical range from which the exponential powers may be selected.

Flowchart 470 continues with analyzing parameters 316, the differential parameters, and the combinatorial parameters to identify a reduced set of parameters correlated with the health state, e.g., in this exemplary method, correlated with hypotension (action 476). Analysis of parameters 316, the differential parameters, and the combinatorial parameters to identify a reduced set of parameters correlated with hypotension can be performed by predictive risk model training software code 110/210a/210b/310, executed by hardware processor 104/204/234/334.

As stated above, parameters 316 and the differential parameters, together, may number in the thousands. As a result, and in light of the process for generating the combinatorial parameters described above, the combination of parameters 316, the differential parameters, and the combinatorial parameters may cumulatively number in the millions. To render analysis of such a large number of variables tractable, in one implementation, analysis of parameters 316, the differential parameters, and the combinatorial parameters may be performed as a receiver operating characteristic (ROC) analysis of those parameters, for example.

ROC analysis is a way to illustrate the performance of a binary classifier as its discrimination threshold is varied. An ROC curve is created by plotting the true positive rate against the false positive rate at various threshold settings. Area under the ROC curve (AUC) can be used to judge the performance of different classifiers, and the higher the AUC is, the better the classifier. On a dataset with positives and negatives of health states predefined, an ROC analysis can be performed for each parameter to obtain its AUC. Then those parameters with large AUC values are retained.

The result of such a ROC analysis is a reduced set of parameters correlated with hypotension. For example, where parameters 316, the differential parameters, and the combinatorial parameters cumulatively number between two and three million, the reduced set of parameters may include less than approximately two hundred parameters identified as being correlated with hypotension.

As shown in Figure 4, flowchart 470 continues with identifying, from among the reduced set of parameters, predictive set of parameters 114/214 enabling prediction of the health state, e.g., hypotension, for a living subject (action 477). Identification of predictive set of parameters 114/214 from the reduced set of parameters may be performed by predictive risk model training software code 110/210a/210b/310, executed by hardware processor 104/204/234/334.

In one implementation, predictive set of parameters 114/214 includes a subset of the reduced set of parameters having the strongest correlation with hypotension. For example, the correlation of each parameter included in the reduced set of parameters may be determined by sequentially testing predictions of the health state, e.g., hypotension, produced using each parameter of the reduced set of parameters. In such an implementation, only those parameters from among the reduced set of parameters having a measured correlation with hypotension that satisfies a threshold or cutoff correlation value is included as one of predictive set of parameters 114/214.

In another implementation, predictive set of parameters 114/214 can be identified using machine learning techniques, such as sequential feature selection, either with forward or backward selection. Using sequential feature selection, the reduced set of parameters are added or removed one by one to a machine learning model: either a classification or a regression model. The sequential feature selection seeks to minimize the mean square error (for regression models) or the misclassification rate (for classification models) over all possible combinations of the reduced set of parameters, by adding (for forward selection) or removing (for backward selection) parameters one by one to/from the regression or classification model. Classification and regression models could include: linear regression, logistic regression, discriminant analysis, neural networks, support vector machines, nearest neighbors, classification and regression trees, or ensemble methods, such as random forests, to name a few examples.

In yet another implementation, predictive set of parameters 114/214 can be identified using machine learning techniques such as Best Subset Selection (leaps and bounds algorithms), Ridge Regression, Lasso Regression, Least Angle Regression, or Principal Components Regression and Partial Least Squares.

As a specific example, where the reduced set of parameters derived from analysis of parameters 316, the differential parameters, and the combinatorial parameters includes up to approximately two hundred parameters, predictive set of parameters 114/214 may number from as little as a few parameters, e.g., five or less, to as many as approximately fifty parameters. An exemplary but non-exhaustive table listing predictive set of parameters 114/214 for the exemplary case of hypotension prediction, as well as exemplary sampling criteria associated with their determination, is provided as Appendix A of the present disclosure. Predictive set of parameters 114/214 may be utilized by predictive risk model training software code 110/210a/210b/310, executed by hardware processor 104/204/234/334, to substantially optimize predictive risk model 112/212 for predicting hypotension for a living subject.

Flowchart 470 can conclude with training predictive risk model 112/212 to include some or all of predictive set of parameters 114/214 using the previously described training subsets (action 478). Predictive risk model 112/212 may include machine learning models: either regression or classification models. Examples of regression and classification models suitable for use in training predictive risk model 112/212 may include linear regression, logistic regression, discriminant analysis, neural networks, support vector machines, nearest neighbors, classification and regression trees, or ensemble methods, such as random forests, to name a few.

Predictive risk model 112/212 may include all or a subset of predictive set of parameters 114/214, as well as additional parameters. Predictive risk model 112/212 may include model coefficients that must be determined by model training. Training the predictive risk model means computing predictive risk model coefficients using numerical procedures to minimize a cost function representing the error of the predictive risk model output to the true value of the training subset.

As an example, a trained predictive risk model from action 478 using logistic regression may be expressed as:

$$\text{Risk Score} = 1/(1 + e^{-A}) \qquad \text{(Equation 2)}$$

Where:

$$A = c_0 + c_1 \times v_1 + c_2 \times v_2 + c_3 \times v_3 + c_4 \times v_4 + c_5 \times v_5 + c_6 \times v_6$$
$$+ c_7 \times v_7^2 \times v_8^2 \times v_9^{-2} + c_8 \times v_2^2 \times v_{10} \times v_{11}^{-1} + c_9 \times \Delta(v_{12}^2 \times v_{13}^2 \times v_{14})$$
$$+ c_{10} \times \Delta(v_{15}^2 \times v_1 \times v_{16}^{-1}) + c_{11} \times \Delta(v_{17}^2 \times v_{18}^2 \times v_{19}^{-2})$$

And where:

$v_1$ = CWI, the cardiac work indexed by patient's body surface area;
$v_2$ = MAPavg, the averaged mean arterial pressure;
$v_3$ = $\Delta$MAPavg, the change of averaged mean arterial pressure when compared to initial values;
$v_4$ = avgSysDec, the averaged pressure at the decay portion of the systolic phase;
$v_5$ = $\Delta$Sys, the change of systolic pressure when compared to initial values;
$v_6$ = ppAreaNor, the normalized area under the arterial pressure waveform;
$v_7$ = biasDia, the bias of the diastolic slope;
$v_8$ = CW, the cardiac work;
$v_9$ = mapDnlocArea, the area under the arterial pressure waveform, between first instance of MAP and the dicrotic notch;
$v_{10}$ = SWcomb, the stroke work;
$v_{11}$ = ppArea, the area under the arterial pressure waveform;
$v_{12}$ = decAreaNor, the normalized area of the decay phase;
$v_{13}$ = slopeSys, the slope of the systolic phase;
$v_{14}$ = Cwk, the Windkessel compliance;
$v_{15}$ = sys_rise_area_nor, the normalized area under the systolic rise phase;
$v_{16}$ = pulsepres, the pulse pressure;
$v_{17}$ = avg_sys, the averaged pressure of the systolic phase;
$v_{18}$ = dpdt2, the maximum value of the second order derivative of the pressure waveform;
$v_{19}$ = dpdt, the maximum value of the first order derivative of the pressure waveform; and
$\Delta$ = the change of the value when compared to its initial value
$c_0$, $c_1$, ..., $c_{11}$ are constant coefficients.

Although not included in flowchart 470, in some implementations the present method may further include updating predictive set of parameters 114/214 and the predictive risk model coefficients based on newly received vital sign data 460. That is to say, hardware processor 104/204/234/334 may be configured to execute predictive risk model training software code 110/210a/210b/310 to update predictive set of parameters 114/214 after the training of predictive risk model 112/212.

Thus, the present application discloses systems and methods for training predictive risk models for a variety of potentially dangerous physiological states to which a critically ill or surgical patient may be susceptible. As discussed above, examples of such physiological states include hypotension, hypovolemia, acute blood loss, septic shock, extubation failure, post-surgical complications, and cardiovascular collapse or crash, to name a few. According to various implementations, the systems and methods disclosed in the present application may be utilized by health care workers to anticipate a dangerous physical state prior to its onset for a living subject. As a result of such forewarning, the systems and methods disclosed in the present application enable preparation of effective medical interventions for administering early treatment of the anticipated condition, or for preventing it entirely.

From the above description it is manifest that various techniques can be used for implementing the concepts described in the present application without departing from the scope of those concepts. Moreover, while the concepts have been described with specific reference to certain implementations, a person of ordinary skill in the art would recognize that changes can be made in form and detail without departing from the scope of those concepts. As such, the described implementations are to be considered in all respects as illustrative and not restrictive. It should also be understood that the present application is not limited to the particular implementations described herein, but many rearrangements, modifications, and substitutions are possible without departing from the scope of the present disclosure.

APPENDIX A

| Description of Predictive Parameters for HypotensionCriteria | Sampling |
| --- | --- |
| 1. TR_bp_dia: Diastolic pressure | 20 sec. average |
| 2. TR_c_wk: The Windkessel Compliance (based on the Langewooters paper) | 20 sec. average |
| 3. TR_CO_disp: Cardiac output | 20 sec. average |
| 4. TR_CO_hsi: Cardiac output computed with a heavily weighted multivariate model based on hyperdynamic conditions | 20 sec. average |
| 5. TR_COaccum_avg: Cardiac output - 5 min. average | 5 min. average |
| 6. TR_dia_area_nodia: Area under the arterial pressure waveform from the dicrotic notch to the start of the next beat with subtracted diastolic pressure | 20 sec. average |
| 7. TR_dpdt_var: Variability in maximum of the first derivative | 20 sec. average |
| 8. TR_dpdt2_var: Variability in maximum of the second derivative | 20 sec. average |
| 9. TR_HR_avg_disp: Heart rate - 5 min. average | 5 min. average |
| 10. TR_K_avg_fp_tp: Multivariate classification model to detect the likelihood of a false positive in the prediction ofK_avg_dm | 20 sec. average |
| 11. TR_t_sys_rise_var: Variability in TR_t_sys_rise | 20 sec. average |
| 12. TR_K_avg_hyp_w: Vascular tone computed from a weighted multivariate model derived from severe hyperdynamic conditions | 20 sec. average |
| 13. TR_K_avg_lco: Multivariate classification model to detect low flow conditions | 20 sec. average |
| 14. TR_kmult: Arterial tone estimate | 20 sec. average |
| 15. TR_kmult_fp_tp: K_avg_fp_tp - 20 sec. Average | 20 sec. average |
| 16. TR_kurt: The kurtosis of the arterial pressure waveform within a beat | 20 sec. average |
| 17. TR_kurt_var: Variability in the kurtosis of the arterial pressure waveform within a beat | 20 sec. average |
| 18. TR_sku: Skewness of the arterial pressure waveform within a beat | 20 sec. average |
| 19. TR_sku_var: Variability in TR_sku | 20 sec. average |
| 20. TR_sku2: Skewness of the 20 sec. reconstructed arterial pressure waveform | 20 sec. average |
| 21. TR_slope_dia: Diastolic slope | 20 sec. average |
| 22. TR_slope_dia_var: Variability in the diastolic slope | 20 sec. average |
| 23. TR_slope_sys: Slope of the systolic rise | 20 sec. average |
| 24. TR_SVV_avg_disp: Stroke volume variation (SVV) - 5 min. average | 5 min. average |
| 25. TR_SVV_disp: SVV sensed by hemodynamic sensor | 20 sec. average |
| 26. TR_SVV_resp: SVV computed with the detection of the respiratory cycles in the signal | 20 sec. average |
| 27. TR_t_dec_var: Variability in time from systolic maximum to start of next heart beat | 20 sec. average |

(continued)

| Description of Predictive Parameters for HypotensionCriteria | Sampling |
|---|---|
| 28. TR_t_sys: Duration of the systolic phase from the start of the heart beat to the dicrotic notch | 20 sec. average |
| 29. TR_t_sys_dec: Time from the systolic maximum to the dicrotic notch | 20 sec. average |
| 30. TR_t_sys_rise: Time from the start of the heart beat to the systolic maximum | 20 sec. average |

The invention further comprises the following embodiments:

1. A system for training a predictive risk model, the system comprising:

a hardware processor and a system memory;

a predictive risk model training software code stored in the system memory;

wherein the hardware processor is configured to execute the predictive risk model training software code to:

receive a vital sign data of each subject of a population of subjects including positive subjects and negative subjects with respect to a health state;
define data sets for use in training the predictive risk model;
transform the vital sign data to a first plurality of parameters characterizing the vital sign data;
obtain a second plurality of differential parameters based on the first plurality of parameters;
generate a third plurality of combinatorial parameters using the first plurality of parameters and the second plurality of differential parameters;
analyze the first plurality of parameters, the second plurality of differential parameters, and the third plurality of combinatorial parameters to identify a reduced set of plurality of parameters correlated with the health state;
identify, from among the reduced set of plurality of parameters, a predictive set of parameters enabling prediction of the health state for a living subject; and
compute predictive risk model coefficients, thereby training the predictive risk model.

2. The system of embodiment 1, wherein each of the third plurality of combinatorial parameters comprises a power combination of a subset of the first plurality of parameters and the second plurality of differential parameters.

3. The system of embodiment 2, wherein the power combination includes integer powers from among negative two, negative one, zero, one, and two (-2, -1, 0, 1, 2).

4. The system of embodiment 1, wherein each of the third plurality of combinatorial parameters comprises a power combination of three parameters from the first plurality of parameters and the second plurality of differential parameters.

5. The system of embodiment 1, wherein the reduced set of plurality of parameters correlated with the health state are identified through a receiver operating characteristic (ROC) analysis of the first plurality of parameters, the second plurality of differential parameters, and the third plurality of combinatorial parameters.

6. The system of embodiment 1, wherein the predictive set of parameters is identified by sequentially testing predictions of the health state produced using each of the reduced set of plurality of parameters.

7. The system of embodiment 1, wherein the vital sign data comprises arterial pressure data, and the health state is hypotension.

8. A method for use by a system for training a predictive risk model, the system including a hardware processor and a predictive risk model training software code stored in a system memory, the method comprising:

receiving, by the predictive risk model training software code executed by the hardware processor, a vital sign data of each subject of a population of subjects including positive subjects and negative subjects with respect to a health state;

defining, by the predictive risk model training software code executed by the hardware processor, data sets for use in training the predictive risk model;

transforming, by the predictive risk model training software code executed by the hardware processor, the vital sign data to a first plurality of parameters characterizing the vital sign data;

obtaining, by the predictive risk model training software code executed by the hardware processor, a second plurality of differential parameters based on the first plurality of parameters;

generating, by the predictive risk model training software code executed by the hardware processor, a third plurality of combinatorial parameters using the first plurality of parameters and the second plurality of differential parameters;

analyzing, by the predictive risk model training software code executed by the hardware processor, the first plurality of parameters, the second plurality of differential parameters, and the third plurality of combinatorial parameters to identify a reduced set of plurality of parameters correlated with the health state;

identifying from among the reduced set of plurality of parameters, by the predictive risk model training software code executed by the hardware processor, a predictive set of parameters enabling prediction of the health state for a living subject; and

computing, by the predictive risk model training software code executed by the hardware processor, predictive risk model coefficients, thereby training the predictive risk model.

9. The method of embodiment 8, wherein each of the third plurality of combinatorial parameters comprises a power combination of a subset of the first plurality of parameters and the second plurality of differential parameters.

10. The method of embodiment 9, wherein the power combination includes integer powers from among negative two, negative one, zero, one, and two (-2, -1, 0, 1, 2).

11. The method of embodiment 8, wherein each of the third plurality of combinatorial parameters comprises a power combination of three parameters from the first plurality of parameters and the second plurality of differential parameters.

12. The method of embodiment 8, wherein analyzing the first plurality of parameters, the second plurality of differential parameters, and the third plurality of combinatorial parameters to identify the reduced set of plurality of parameters correlated with the health state includes performing a receiver operating characteristic (ROC) analysis of the first plurality of parameters, the second plurality of differential parameters, and the third plurality of combinatorial parameters.

13. The method of embodiment 8, wherein identifying the predictive set of parameters includes sequentially testing predictions of the health state produced using each of the reduced set of plurality of parameters.

14. The method of embodiment 8, wherein the vital sign data comprises arterial pressure data, and the health state is hypotension.

15. A computer-readable non-transitory medium having stored thereon instructions, which when executed by a hardware processor, instantiate a method comprising:

receiving a vital sign data of each subject of a population of subjects including positive subjects and negative subjects with respect to a health state;

defining data sets for use in training a predictive risk model;

transforming the vital sign data to a first plurality of parameters characterizing the vital sign data;

obtaining a second plurality of differential parameters based on the first plurality of parameters;

generating a third plurality of combinatorial parameters using the first plurality of parameters and the second plurality of differential parameters;

analyzing the first plurality of parameters, the second plurality of differential parameters, and the third plurality of combinatorial parameters to identify a reduced set of plurality of parameters correlated with the health state;

identifying, from among the reduced set of plurality of parameters, a predictive set of parameters enabling prediction of the health state for a living subject; and

computing predictive risk model coefficients, thereby training the predictive risk model.

16. The computer-readable non-transitory medium of embodiment 15, wherein each of the third plurality of combinatorial parameters comprises a power combination of a subset of the first plurality of parameters and the second plurality of differential parameters.

17. The computer-readable non-transitory medium of embodiment 16, wherein the power combination includes integer powers from among negative two, negative one, zero, one, and two (-2, -1, 0, 1, 2).

18. The computer-readable non-transitory medium of embodiment 15, wherein each of the third plurality of combinatorial parameters comprises a power combination of three parameters from the first plurality of parameters and the second plurality of differential parameters.

19. The computer-readable non-transitory medium of embodiment 18, wherein analyzing the first plurality of parameters, the second plurality of differential parameters, and the third plurality of combinatorial parameters to identify the reduced set of plurality of parameters correlated with the health state includes performing a receiver operating characteristic (ROC) analysis of the first plurality of parameters, the second plurality of differential parameters, and the third plurality of combinatorial parameters.

20. The computer-readable non-transitory medium of embodiment 15, wherein identifying the predictive set of parameters includes sequentially testing predictions of the health state produced using each of the reduced set of plurality of parameters.

## Claims

1.  A system (102; 202; 230; 330) for training a predictive risk model (112; 212) utilized by health care workers as a forewarning to anticipate a dangerous physiological state prior to its onset, the system comprising:

    a hardware processor (104; 204, 234; 334) and a system memory (106);
    a predictive risk model training software code (110; 210a; 210b; 310) stored in the system memory (106);
    wherein the hardware processor (104; 204; 234; 334) is configured to execute the predictive risk model training software code (110) to:

    receive a vital sign data (160) comprising hemodynamic data corresponding to an arterial pressure of each subject of a population of subjects including positive subjects (150) and negative subjects (154) with respect to a health state, said health state being hypotension;
    define data sets for use in training the predictive risk model (112), wherein the data sets include:

    (i) a positive training subset comprising vital sign data (160) of the positive subjects (150) in the population of subjects, said vital sign data (160) obtained (a) at a time period when the hypotension occurred and (b) at predetermined times prior to the occurrence of the hypotension;
    (ii) a negative training subset comprising vital sign data (160) obtained (a) of the negative subjects (154) in the population of subjects at a time period when the hypotension did not occur and (b) obtained of the positive subjects (150) in the population of subjects at a time period when the hypotension did not occur, wherein said time period shall be distanced by a predetermined period of time from the time period when the hypotension occurred;

    transform the vital sign data (160) to a first plurality of parameters (316) characterizing the vital sign data (160);
    obtain a second plurality of differential parameters based on the first plurality of parameters (316);
    generate a third plurality of combinatorial parameters using the first plurality of parameters (316) and the second plurality of differential parameters;
    analyze the first plurality of parameters (316), the second plurality of differential parameters, and the third plurality of combinatorial parameters to identify a reduced set of plurality of parameters correlated with the hypotension;
    identify, from among the reduced set of plurality of parameters, a predictive set of parameters (114; 214) enabling prediction of the hypotension for a living subject;
    compute predictive risk model coefficients to minimize a cost function representing an error of a predictive risk model output, thereby training the predictive risk model (112; 212); and
    utilizing the predictive risk model (112; 212) as a forewarning to anticipate a dangerous physiological state prior to its onset.

2. The system (102; 202; 230; 330) of claim 1, wherein each of the third plurality of combinatorial parameters comprises a power combination of a subset of the first plurality of parameters (316) and the second plurality of differential parameters.

3. The system (102; 202; 230; 330) of claim 2, wherein the power combination includes integer powers from among negative two, negative one, zero, one, and two (-2, -1, 0, 1,2).

4. The system (102; 202; 230; 330) of claim 1, wherein each of the third plurality of combinatorial parameters comprises a power combination of three parameters from the first plurality of parameters and the second plurality of differential parameters.

5. The system (102; 202; 230; 330) of any one of claims 1 to 4, wherein the reduced set of plurality of parameters correlated with the hypotension are identified through a receiver operating characteristic (ROC) analysis of the first plurality of parameters (316), the second plurality of differential parameters, and the third plurality of combinatorial parameters.

6. The system (102; 202; 230; 330) of any one of claims 1 to 5, wherein the predictive set of parameters (114; 214) is identified by sequentially testing predictions of the hypotension produced using each of the reduced set of plurality of parameters.

7. The system (102; 202; 230; 330) of claim 6, wherein the predictive set of parameters (114; 214) is identified by having a measured correlation with hypotension that satisfies a threshold correlation value.

8. The system (102; 202; 230; 330) of claim 6, wherein the predictive set of parameters (114; 214) is identified by adding the parameters of the reduced set of parameters one by one to a classification model or a regression model, or removing parameters of the reduced set of parameters one by one from the classification model or the regression model.

9. The system (102; 202; 230; 330) of any of claims 1 to 8, wherein the hardware processor (104; 204, 234; 334) transforms the vital sign data (160) by executing the predictive risk model training software code (110; 210a; 210b; 310) to:
determine, from the vital sign data (160), on a heartbeat-by-heartbeat basis, indicia representative of one or more of:

   start of a heartbeat;
   maximum systolic pressure marking end of systolic rise;
   presence of a dicrotic notch marking end of systolic decay;
   diastole of the heartbeat; and
   slopes of an arterial pressure waveform.

10. The system (102; 202; 230; 330) of any one of claims 1 to 9, wherein the plurality of first parameters (316) includes at least of one of:

   cardiac output;
   cardiac index;
   stroke volume;
   stroke volume index;
   pulse rate;
   systemic vascular resistance;
   systemic vascular resistance index;
   mean arterial pressure (MAP);
   baroreflex sensitivity measures;
   hemodynamic complexity measures; and
   frequency domain hemodynamic features.

11. The system (102; 202; 230; 330) of any one of claims 1 to 10, wherein the hardware processor (104; 204, 234; 334) is configured to transmit the predictive risk model via a communication network (120) to a client system (130).

12. The system (102; 202; 230; 330) of claim 11, wherein the client system (130) is a mobile communication device.

**13.** The system (102; 202; 230; 330) of any one of claims 1 to 12, wherein the system (102; 202; 230; 330) includes a display (232), and the hardware processor (104; 204, 234; 334) is configured to show the vital sign data (160) on the display (232).

**14.** A method for use by a system according to claim 1, the method comprising:

receiving a vital sign data (160) comprising hemodynamic data corresponding to an arterial pressure of each subject of a population of subjects including positive subjects (150) and negative subjects (154) with respect to a health state said health state being hypotension;

defining data sets for use in training the predictive risk model (112), wherein the data sets include:

(i) a positive training subset comprising vital sign data (160) of the positive subjects (150) in the population of subjects, said vital sign data (160) obtained (a) at a time period when the hypotension occurred and (b) at predetermined times prior to the occurrence of the hypotension;

(ii) a negative training subset comprising vital sign data (160) obtained (a) of the negative subjects (154) in the population of subjects at a time period when the hypotension did not occur and (b) obtained of the positive subjects (150) in the population of subjects at a time period when the hypotension did not occur, wherein said time period shall be distanced by a predetermined period of time from the time period when the hypotension occurred;

transforming the vital sign data (160) to a first plurality of parameters (316) characterizing the vital sign data (160);

obtaining a second plurality of differential parameters based on the first plurality of parameters (316);

generating a third plurality of combinatorial parameters using the first plurality of parameters (316) and the second plurality of differential parameters;

analyzing the first plurality of parameters (316), the second plurality of differential parameters, and the third plurality of combinatorial parameters to identify a reduced set of plurality of parameters correlated with the hypotension;

identifying from among the reduced set of plurality of parameters a predictive set of parameters (114; 214) enabling prediction of the hypotension for a living subject;

computing predictive risk model coefficients to minimize a cost function representing an error of a predictive risk model output, thereby training the predictive risk model (112; 212); and

utilizing the predictive risk model (112; 212) as a forewarning to anticipate a dangerous physiological state prior to its onset.

**15.** A computer-readable non-transitory medium having stored thereon instructions, which when executed by a hardware processor, instantiates the method according to claim 14.

# Fig. 1

100

System 102

Hardware Processor 104

System Memory 106

110 — Predictive Risk Model Training Software Code

112 — Predictive Risk Model

Predictive Set of Parameters 114

160

122

160

Network 120

160

160

Positive Subject Population

150

Negative Subject Population

154

140

130

# Fig. 2

200

**System 202**

Hardware Processor ~ 204

**System Memory 206**

Predictive Risk Model Training Software Code ~ 210a

222

Display ~ 232

**Client System 230**

Client Hardware Processor ~ 234

**Client System Memory 236**

Predictive Risk Model Training Software Code ~ 210b

Predictive Risk Model

Predictive Set of Parameters 214

212

# Fig. 3

330

332

Pressure (mmHg)

364
360
358
m
366
362
368

316

Time (s)

Hardware Processor 334

336
System Memory

338

Computer-Readable
Non-Transitory
Medium

Predictive Risk
Model Training
Software Code

318

310

**Fig. 4**

Receive vital sign data of each subject of a
population of subjects including positive
subjects and negative subjects with respect
to a health state ⌐471

470

Define data sets for use in training a
predictive risk model ⌐472

Transform the vital sign data to parameters
characterizing the vital sign data ⌐473

Obtain differential parameters based
on the parameters transformed from the
vital sign data ⌐474

Generate combinatorial parameters using the
parameters transformed from the vital sign
data and the differential parameters ⌐475

Analyze the parameters transformed from the
vital sign data, the differential parameters,
and the combinatorial parameters to identify
a reduced set of plurality of parameters
correlated with the health state ⌐476

Identify, from the reduced set of plurality of
parameters, a predictive set of parameters
enabling prediction of the health state for a
living subject ⌐477

Compute predictive risk model coefficients,
thereby training the predictive risk model ⌐478